(19) Europäisches Patentamt
European Patent Office
Office européen des brevets

(11) EP 1 889 620 A1

(12) # EUROPEAN PATENT APPLICATION
published in accordance with Art. 153(4) EPC

(43) Date of publication:
**20.02.2008 Bulletin 2008/08**

(21) Application number: **06757199.2**

(22) Date of filing: **09.06.2006**

(51) Int Cl.:
***A61K 31/44*** *(2006.01)* ***A61K 9/08*** *(2006.01)*
***A61K 47/32*** *(2006.01)* ***A61K 47/34*** *(2006.01)*
***A61K 47/38*** *(2006.01)* ***A61P 27/02*** *(2006.01)*

(86) International application number:
**PCT/JP2006/311576**

(87) International publication number:
**WO 2006/132342 (14.12.2006 Gazette 2006/50)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **09.06.2005 JP 2005169015**

(71) Applicant: **Santen Pharmaceutical Co., Ltd**
**Osaka-shi, Osaka 533-8651 (JP)**

(72) Inventors:
- **ENDO, Yoko**
  **c/o Santen Pharmaceutical Co., Ltd.**
  **Osaka-shi, Osaka 5338651 (JP)**
- **KIMURA, Akio**
  **c/o Santen Pharmaceutical Co., Ltd.**
  **Osaka-shi, Osaka 5338651 (JP)**

(74) Representative: **Müller-Boré & Partner**
**Patentanwälte**
**Grafinger Straße 2**
**81671 München (DE)**

(54) **EYE DROP CONTAINING ROFLUMILAST**

(57) An object of the present invention is to enhance the efficacy of roflumilast in an eye drop containing roflumilast as an active ingredient. By formulating at least one type of viscosity-increasing agent in the eye drop containing roflumilast as an active ingredient, an eye drop in which the efficacy of roflumilast is enhanced can be prepared.

EP 1 889 620 A1

## Description

Technical Field

The present invention relates to an eye drop containing roflumilast as an active ingredient, in which the efficacy of roflumilast is enhanced.

Background Art

Roflumilast has the chemical name of N-(3,5-dichloropyridin-4-yl)-3-cyclopropylmethoxy-4-difluoromethoxy benzamide and is represented by the following chemical structural formula.

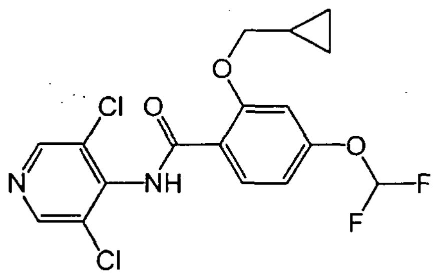

Roflumilast is useful in the mediation or inhibition of enzymatic activity of phosphodiesterase IV. In JP-T-8-512041, roflumilast is known to be useful as a therapeutic agent for allergic and inflammatory diseases, particularly asthma and bronchitis, and it is suggested that roflumilast is effective also in the treatment of allergic and inflammatory diseases in eyes. In WO 03/099278, an ophthalmic ointment, an eye drop such as a suspension-type eye drop and an ophthalmic oral preparation are described as an ophthalmic preparation containing roflumilast as an active ingredient.

On the other hand, a polymer compound such as polyvinyl alcohol, carboxyvinyl polymer or hydroxypropylmethyl cellulose is used as a viscosity-increasing agent in the field of eye drops. In WO 03/099278 and WO 03/099334, several types of cellulose derivatives and vinyl polymer compounds are described as a suspension stabilizer for a suspension-type eye drop containing roflumilast as an active ingredient.

However, there is no description in any of the above publications suggesting that the efficacy of roflumilast can be enhanced by formulating a viscosity-increasing agent in an eye drop containing roflumilast as an active ingredient.

Disclosure of the invention

Problems to be Solved

It is considered that phosphodiesterase IV inhibitors are useful for the treatment of eye diseases such as allergic conjunctivitis, vernal kerato conjunctivitis and blepharitis from the above-mentioned effect. Among the inhibitors, roflumilast has a high phosphodiesterase IV inhibitory activity. Therefore, roflumilast is expected as a new therapeutic agent for the above-mentioned eye diseases. Thus, it is an interesting subject to further enhance the efficacy of roflumilast.

It has been reported that when such a phosphodiesterase IV inhibitor is used at a high dose, it causes a side effect such as nausea, vomiting, headache or diarrhea, and there is a problem that the clinical usefulness thereof is limited because of this side effect. This problem also applies to roflumilast having the same inhibitory effect. Further, in the case where it is applied to an eye disease, an ophthalmic preparation such as an eye drop or an ophthalmic ointment is commonly used. However, because such a preparation is generally administered at frequent times, this problem of side effect cannot be disregarded. Accordingly, it is considered that if the efficacy of roflumilast can be enhanced, the amount of roflumilast can be reduced while maintaining a desired efficacy and the side effect can be avoided or reduced to the minimum. Thus, also from the viewpoint of avoiding a side effect, it is an interesting subject to enhance the efficacy of roflumilast.

On the other hand, because roflumilast is hardly soluble, generally there is a limitation on the applicable dosage form of an ophthalmic preparation, and the dosage form of an ophthalmic ointment or a suspension-type eye drop can be contemplated. However, although an ophthalmic ointment or a suspension-type eye drop is commonly used as an ophthalmic preparation, the ophthalmic ointment is remarkably sticky and is not preferred in terms of the usability, and in the suspension-type eye drop, because a drug precipitates when the suspension-type eye drop is stored still, it is necessary to sufficiently shake it before use to obtain a uniform dispersion, therefore, it is not preferred in terms of the

convenience. In addition, it cannot be denied that there is a possibility that a problem of aggregation or caking of drug occurs in the suspension-type eye drop. Accordingly, it is a big subject to prepare a soluble-type eye drop.

Means of Solving Problems

Thus, the present inventors made intensive studies of an eye drop as described above and prepared and examined various eye drops containing roflumilast in which a widely used additive was formulated. As a result, they surprisingly found that by formulating a viscosity-increasing agent such as a vinyl polymer compound or a cellulose derivative, the efficacy of roflumilast can be enhanced. This is a surprising finding from the viewpoint that although it is known that the persistence of drug efficacy is improved by formulating a viscosity-increasing agent, it is totally unknown that the drug efficacy itself is enhanced by formulating a viscosity-increasing agent. Further, according to the present invention, the concentration of roflumilast can be decreased while maintaining the efficacy of roflumilast, therefore, a side effect caused by a phosphodiesterase IV inhibitory effect can be avoided or reduced to the minimum.

Further, it was also found that by making the eye drop containing roflumilast a soluble type by formulating a surfactant therein, the efficacy of roflumilast can be more effectively enhanced. According to the present invention, even if an eye drop contains insoluble roflumilast, a stable soluble-type eye drop can be prepared while maintaining a desired efficacy, and an eye drop which is superior to conventional ones in terms of usability and convenience can be provided. That is, the present invention relates to

(1) an eye drop comprising roflumilast as an active ingredient, wherein by formulating at least one type of viscosity-increasing agent therein, the efficacy of roflumilast is enhanced and also the viscosity of the eye drop at 25°C is made 1.5 to 500 mPa·s;
(2) the eye drop according to the above (1), wherein the viscosity of the eye drop at 25°C is 1.5 to 50 mPa·s;
(3) the eye drop according to the above (1), wherein the concentration of roflumilast is 0.00001 to 0.05% (w/v);
(4) the eye drop according to the above (1), wherein the concentration of roflumilast is 0.0001 to 0.03% (w/v);
(5) the eye drop according to the above (1), wherein the viscosity-increasing agent is hydroxypropylmethyl cellulose, polyvinyl alcohol or carboxyvinyl polymer;
(6) the eye drop according to the above (1), wherein it is made a soluble type by further formulating a surfactant therein;
(7) the eye drop according to the above (6), wherein the surfactant is at least one member selected from polysorbate 80, polyoxyethylene hydrogenated castor oil 60, polyoxyl 35 castor oil and polyoxyl 40 monostearate; and
(8) a method in which in an eye drop comprising roflumilast as an active ingredient, by formulating at least one type of viscosity-increasing agent therein, the efficacy of roflumilast is enhanced and also the viscosity of the eye drop at 25°C is made 1.5 to 500 mPa·s.

The concentration of roflumilast which is an active ingredient of the eye drop according to the present invention may be a concentration that allows its therapeutic efficacy to be exhibited and also a soluble-type eye drop to be obtained, however, it is preferably 0.00001 to 0.05% (w/v), and more preferably 0.0001 to 0.03% (w/v).

Examples of the viscosity-increasing agent to be used in the present invention include vinyl polymer compounds such as polyvinyl alcohol, polyvinylpyrrolidone, carboxyvinyl polymer and polyvinylmethyl ether; cellulose derivatives such as hydroxypropylmethyl cellulose, hydroxypropylethyl cellulose, hydroxypropyl cellulose, hydroxyethyl cellulose, methyl cellulose, ethylmethyl cellulose, ethylpropyl cellulose, carboxymethyl cellulose or salts thereof and carboxypropyl cellulose or salts thereof; polysaccharides such as hyaluronic acid or salts thereof, dextran and cyclodextrin; and polymer compounds such as sodium polyacrylate and sodium chondroitin sulfate. Preferred examples of the viscosity-increasing agent include hydroxypropylmethyl cellulose, polyvinyl alcohol and carboxyvinyl polymer.

In the present invention, the amount of the viscosity-increasing agent cannot be definitely defined because it varies depending on the molecular weight or type of the viscosity-increasing agent. It is preferred that the amount of the viscosity-increasing agent is appropriately selected such that the viscosity of the eye drop of the present invention falls within the range of 1.5 to 500 mPa·s using an E-type viscometer (at 25°C and a shear rate of 100 $s^{-1}$). This is because when the viscosity of the eye drop is 1.5 mPa·s or higher, the efficacy of roflumilast can be more effectively enhanced; and when the viscosity of the eye drop is too high, it is not preferred from the viewpoint of usability that the resulting eye drop becomes sticky or it becomes difficult to instill the eye drop and from the viewpoint of production that it becomes difficult to perform a filtration step for a sterilization treatment of the eye drop. A more preferred viscosity of the eye drop is 1.5 to 50 mPa·s.

The viscosity of the eye drop of the present invention is measured using a rotational viscometer RS 100 (HAAKE Co.) which is an E-type viscometer, and is represented by a value at a measurement temperature of 25°C and a shear rate of 100 $s^{-1}$ when the shear rate is increased in the range from 0.3 $s^{-1}$ to 200 $s^{-1}$.

The "soluble type" of the soluble-type eye drop of the present invention refers to a state in which roflumilast is dissolved in a liquid solvent of an eye drop.

The eye drop according to the present invention may be made a soluble type by further formulating a surfactant therein. The surfactant improves the solubility in water of roflumilast thereby to make the eye drop containing roflumilast a soluble type and more effectively enhance the efficacy of roflumilast in the eye drop. Specific examples of the surfactant include polyoxyethylene fatty acid esters such as polysorbate 80 [polyoxyethylene sorbitan monooleate], polysorbate 60 [polyoxyethylene sorbitan monostearate], polysorbate 40 [polyoxyethylene sorbitan monopalmitate], polyoxyethylene sorbitan monolaurate, polyoxyethylene sorbitan trioleate and polysorbate 65 [polyoxyethylene sorbitan tristearate]; polyoxyethylene castor oil derivatives such as polyoxyethylene hydrogenated castor oil 10, polyoxyethylene hydrogenated castor oil 40, polyoxyethylene hydrogenated castor oil 50, polyoxyethylene hydrogenated castor oil 60, polyoxyl 5 castor oil, polyoxyl 9 castor oil, polyoxyl 15 castor oil, polyoxyl 35 castor oil and polyoxyl 40 castor oil; polyoxyethylene polyoxypropylene glycols such as polyoxyethylene (160) polyoxypropylene (30) glycol [Pluronic F68], polyoxyethylene (42) polyoxypropylene (67) glycol [Pluronic P123], polyoxyethylene (54) polyoxypropylene (39) glycol [Pluronic P85], polyoxyethylene (196) polyoxypropylene (67) glycol [Pluronic F127] and polyoxyethylene (20) polyoxypropylene (20) glycol [Pluronic L-44]; polyoxyl 40 stearate, sucrose fatty acid esters and the like. Preferred examples thereof include polysorbate 80 [polyoxyethylene sorbitan monooleate], polyoxyethylene hydrogenated castor oil 60, polyoxyl 35 castor oil, polyoxyl 40 stearate and the like. These surfactants can be used alone or in combination of two or more types. Particularly preferred examples of the surfactant include polysorbate 80 [polyoxyethylene sorbitan monooleate] or polyoxyl 35 castor oil, both of which are commonly used as an additive for an eye drop.

The amount of the surfactant can be appropriately increased or decreased according to the concentration of roflumilast in the eye drop of the present invention, and is preferably an amount which allows roflumilast to dissolve in the eye drop. For example, if the surfactant is polyoxyl 35 castor oil, the amount thereof is preferably 1 to 10% (w/v), more preferably 2 to 6% (w/v). Further, if the surfactant is polysorbate 80, the amount thereof is preferably 1 to 10% (w/v), more preferably 2 to 6% (w/v).

The eye drop of the present invention can be prepared by a widely used method, and a tonicity agent, a buffer, a pH adjusting agent, a stabilizer, a preservative or the like can be added thereto as needed.

Examples of the tonicity agent include glycerin, propylene glycol, polyethylene glycol, trehalose, sucrose, sorbitol, mannitol, sodium chloride, potassium chloride, calcium chloride, magnesium chloride and the like.

Examples of the buffer include phosphoric acid, phosphate, boric acid, borax, citric acid, acetic acid, ε-aminocaproic acid, trometamol and the like.

Examples of the pH adjusting agent include hydrochloric acid, citric acid, phosphoric acid, acetic acid, sodium hydroxide, potassium hydroxide, boric acid, borax, sodium carbonate, sodium hydrogen carbonate and the like.

Examples of the stabilizer include edetic acid, sodium edetate and the like.

Examples of the preservative include, commonly used sorbic acid, potassium sorbate, benzalkonium chloride, benzethonium chloride, methyl p-hydroxybenzoate, ethyl p-hydroxybenzoate, propyl p-hydroxybenzoate, butyl p-hydroxybenzoate, chlorhexidine gluconate, chlorobutanol and the like. These preservatives can also be used in combination.

The pH of the eye drop of the present invention is preferably adjusted to 4.0 to 8.5, and the osmotic pressure ratio thereof is preferably adjusted to about 1.0.

The eye disease for which the eye drop of the present invention is used is a disease associated with an allergy or inflammation. Examples thereof include allergic conjunctivitis, vernal keratoconjunctivitis, atopic keratoconjunctivitis, infectious keratoconjunctivitis, blepharitis, pruritus accompanying ophthalmic surgery such as cataract surgery, keratoconjunctivitis sicca, uveitis, age-related macular degeneration, diabetic retinopathy and the like.

The present invention also relates to a method of treating an eye disease comprising administering a pharmacologically effective amount of an eye drop containing roflumilast as an active ingredient, in which by formulating at least one type of viscosity-increasing agent therein, the efficacy of roflumilast is enhanced and also the viscosity of the eye drop at 25°C is made 1.5 to 500 mPa·s to a patient.

The instillation times of the eye drop of the present invention can be appropriately selected depending on the symptom, age, dosage form and the like, however, the eye drop may be instilled once to several times (for example, 1 to 6 times) a day in an amount of one to several drops at a time.

Advantage of the Invention

As will be described in detail in the section of Pharmacological Test mentioned below, an apparently more excellent efficacy is observed in the eye drop of the present invention in which a viscosity-increasing agent such as hydroxypropylmethyl cellulose, polyvinyl alcohol or carboxyvinyl polymer is formulated compared with a comparative example in which a viscosity-increasing agent is not formulated. That is, by formulating at least one type of viscosity-increasing agent, the efficacy of roflumilast can be enhanced. Further, in the eye drop of the present invention, the concentration of roflumilast can be lowered while maintaining a desired efficacy. Therefore, a side effect of a phosphodiesterase IV inhibitor can be avoided or reduced to the minimum. Further, by formulating a surfactant therein, a soluble type of eye drop containing roflumilast can be prepared, and an eye drop which is superior to conventional ones in terms

of usability and convenience can be provided.

Best Mode for Carrying Out the Invention

Hereinafter, a pharmacological test and preparation examples will be shown, however, these examples are for understanding the present invention well, and are not meant to limit the scope of the present invention.

[Pharmacological Test]

Test for inhibition of conjunctivitis symptom using allergic conjunctivitis model

By using an allergic conjunctivitis model, a test for inhibition of conjunctivitis symptom (edema and hyperemia) of N-(3,5-dichloro-4-pyridyl)-3-chloropropylmethoxy-4-difluoromethoxy benzamide (roflumilast) was studied.

1. Test 1

The concentration of roflumilast was kept at a constant level, and an effect of formulating of a viscosity-increasing agent on an allergic conjunctivitis inhibitory effect was studied.

1-1. Preparation of test solutions

Example 1

Polyoxyl 35 castor oil (5 g, a surfactant) and roflumilast (0.01 g) were put in a 100 mL flask, and purified water, hydroxypropylmethyl cellulose 2906 (trade name: Metolose 65SH-4000, manufactured by Shin-Etsu Chemical Co., Ltd.) (0.5 g), dibasic sodium phosphate (q.s.) and sodium chloride (q.s.) were added thereto and the mixture was stirred to dissolve the ingredients. Thereafter, the pH of the solution was adjusted to 7 with sodium hydroxide or dilute hydrochloric acid, and purified water was added thereto to make the total volume 100 mL, whereby a clear eye drop (viscosity: 11.7 mPa·s) was obtained. Hereinafter the resulting solution is designated as Test solution 1.

Example 2

A clear eye drop (viscosity: 1.7 mPa·s) was obtained by carrying out the same procedure as described in Example 1 except that a 5% aqueous solution of polyvinyl alcohol (20 g) was used instead of hydroxypropylmethyl cellulose (0.5 g). Hereinafter the resulting solution is designated as Test solution 2. Incidentally, the 5% aqueous solution of polyvinyl alcohol was obtained as follows. Polyvinyl alcohol (trade name: KURARAY POVAL PVA-205, manufactured by KURARAY CO., LTD.) (5 g) was put in a 100 mL flask, 80 mL of purified water was added thereto, the mixture was heated to dissolve polyvinyl alcohol at about 60°C, the resulting solution was cooled while stirring with a magnetic stirrer, and purified water was added thereto to make the total volume 100 mL.

Comparative Example 1

A clear eye drop (viscosity: 1.2 mPa·s) was obtained by carrying out the same procedure as described in Example 1 except that hydroxypropylmethyl cellulose (0.5 g) was not added. Hereinafter the resulting solution is designated as Comparative solution 1.

The Test solutions and Comparative solution prepared by the above-mentioned procedures are shown in Table 1, respectively. For the sake of simplifying the table, only roflumilast and a viscosity-increasing agent both of which are necessary components for the comparison and evaluation of the effect of the present invention are shown in the formulation in Table 1.

[Table 1]

Table 1

| Component | concentration(% (w/v)) | | |
|---|---|---|---|
| | Test solution 1 | Test solution 2 | Comparative solution 1 |
| Roflumilast | 0.01 | 0.01 | 0.01 |
| Hydroxypropylmethyl cellulose | 0.5 | | |

(continued)

| Component | concentration(% (w/v)) | | |
|---|---|---|---|
| | Test solution 1 | Test solution 2 | Comparative solution 1 |
| Polyvinyl alcohol | | 1 | |
| pH | 7 | 7 | 7 |
| Appearance | Clear | Clear | Clear |
| Viscosity (mPa·s) | 11.7 | 1.7 | 1.2 |

1-2. Test Method

Ovalbumin adsorbed to aluminum hydroxide gel (20 μg/mL) was suspended in a physiological saline solution, and 100 μL each of the resulting suspension was subconjunctivally injected into both eyes of male Hartley guinea pigs at 5 weeks of age, whereby active sensitization was carried out. On day 15 after the sensitization, a physiological saline solution containing 0.05% (w/v) ovalbumin was instilled into both eyes at a dose of 10 μL/eye, and on day 22 after the sensitization, a physiological saline solution containing 0.5% (w/v) ovalbumin was instilled into both eyes at a dose of 10 μL/eye, whereby allergic conjunctivitis was induced.

At 30 minutes before the instillation of ovalbumin on day 22 after the sensitization, any of the Test solutions and Comparative solution was instilled into both eyes of the above-mentioned guinea pigs at a dose of 10 μL/eye. Incidentally, as a control, a physiological saline solution containing 0.001% (w/v) hydroxypropylmethyl cellulose (trade name: TC5R, manufactured by Shin-Etsu Chemical Co., Ltd.) was instilled into both eyes of the above-mentioned guinea pigs at a dose of 10 μL/eye.

1-3. Evaluation Method

At 30 minutes after the instillation of ovalbumin on day 22 after the sensitization, the conjunctivitis symptom of the guinea pigs was scored in accordance with the following evaluation criteria (Table 2) and evaluated.

Inhibition ratio = 100 − ([average value of scores of conjunctivitis symptom for Test solution or Comparative solution] / [average value of scores of conjunctivitis symptom for control] × 100)

[Table 2]

Table 2

| Score | Conditions of conjunctival edema and hyperemia |
|---|---|
| 0 | The eyelids are open, and edema and hyperemia are not observed at all in the conjunctiva. |
| 0.5 | The eyelids are open, and slight edema and hyperemia are observed in the conjunctiva. |
| 1 | The eyelids are open, and mild edema and hyperemia are observed in the upper and lower conjunctivae. |
| 2 | The upper and lower eyelid margins are spaced a little from the eyeball, and apparent edema and hyperemia are observed when the eyelids are open. |
| 3 | The upper and lower eyelid margins are spaced sufficiently from the eyeball, and edema covers a part of the cornea even if the eyelids are not opened. |
| 4 | The upper and lower eyelid margins are spaced sufficiently from the eyeball, and edema covers a half or more of the cornea even if the eyelids are not opened. |

The inhibition ratios of Test solution 1, Test solution 2 and Comparative solution 1 are shown in Table 3 (the case number was 16 or 18 eyes).

[Table 3]

Table 3

| | Inhibition ratio (%) |
|---|---|
| Test solution 1 | 22.2 |
| Test solution 2 | 18.5 |
| Comparative solution 1 | 0 |

1-4. Test Results and Discussion

As is apparent from Table 3, a significant difference in the efficacy of roflumilast was observed between the Test solutions (the eye drops of the present invention) in which a viscosity-increasing agent was formulated and the Comparative solution in which a viscosity-increasing agent was not formulated although the same amount of roflumilast was formulated therein. Accordingly, it was confirmed that the inhibitory effect of roflumilast on allergic conjunctivitis symptom is enhanced by formulating a viscosity-increasing agent in an eye drop containing roflumilast as an active ingredient.

2. Test 2

An allergic conjunctivitis inhibitory effect of formulating of a viscosity-increasing agent when the concentration of roflumilast was changed was studied.

2-1. Preparation of test solutions

Example 3

Polyoxyl 35 castor oil (5 g, a surfactant) and roflumilast (0.01 g) were put in a 100 mL flask, and purified water and carboxyvinyl polymer (0.3 g) were added thereto and the mixture was stirred to dissolve the ingredients. Thereafter, the pH of the solution was adjusted to 7 with sodium hydroxide or dilute hydrochloric acid, and purified water was added thereto to make the total volume 100 mL, whereby a clear eye drop (viscosity: 453.8 mPa·s) was obtained. Hereinafter the resulting solution is designated as Test solution 3.

Example 4

Polyoxyl 35 castor oil (5 g, a surfactant) and roflumilast (0.01 g) were put in a 100 mL flask, and purified water, a 5% aqueous solution of polyvinyl alcohol (36 g), concentrated glycerin (q.s.) and borax (q.s.) were added thereto and the mixture was stirred to dissolve the ingredients. Thereafter, the pH of the solution was adjusted to 8 with sodium hydroxide or dilute hydrochloric acid, and purified water was added thereto to make the total volume 100 mL, whereby a clear eye drop (viscosity: 2.4 mPa·s) was obtained. Hereinafter the resulting solution is designated as Test solution 4.

Comparative Example 2

Polysorbate 80 (0.005 g, a surfactant) and roflumilast (0.1 g) were put in a 100 mL flask, and purified water and hydroxypropylmethyl cellulose (trade name: TC5R, manufactured by Shin-Etsu Chemical Co., Ltd.) (0.005 g) were added thereto and the mixture was stirred to dissolve the ingredients. Thereafter, the pH of the solution was adjusted to 7 with sodium hydroxide or dilute hydrochloric acid, and purified water was added thereto to make the total volume 100 mL, whereby a suspension-type eye drop (viscosity: <1.0 mPa·s) was obtained. Hereinafter the resulting solution is designated as Comparative solution 2.

The Test solutions and Comparative solution prepared by the above-mentioned procedures are shown in Table 4, respectively. For the sake of simplifying the table, only roflumilast and a viscosity-increasing agent both of which are necessary components for the comparison and evaluation of the effect of the present invention are shown in the formulation in Table 4.

[Table 4]

Table 4

| Component | concentration (% (w/v)) | | |
|---|---|---|---|
| | Test solution 3 | Test solution 4 | Comparative solution 2 |
| Roflumilast | 0.01 | 0.01 | 0.1 |
| Carboxyvinyl polymer | 0.3 | | |
| Polyvinyl alcohol | | 1.8 | |
| Hydroxypropylmethyl cellulose | | | 0.001 |
| pH | 7 | 8 | 7 |
| Appearance | Clear | Clear | Suspended |
| Viscosity (mPa·s) | 453.8 | 2.4 | < 1.0 |

2-2. Test Method and Evaluation Method

The test was carried out in the same manner as the method described in 1-2. As a control for each test solution, one obtained by eliminating only roflumilast from each test solution was used. The evaluation was carried out by the same method as described in 1-3. The inhibition ratios (%) of Test solutions and Comparative solution are shown in Table 5 (the case number was 16 or 18 eyes).

[Table 5]

Table 5

| | Inhibition ratio (%) |
|---|---|
| Test solution 3 | 35.5 |
| Test solution 4 | 33.3 |
| Comparative solution 2 | 33.3 |

2-3. Test Results and Discussion

As is apparent from Table 5, the soluble-type eye drop obtained by formulating a viscosity-increasing agent in an eye drop containing 0.01% roflumilast as an active ingredient showed an inhibition ratio equivalent to that of the suspension-type eye drop containing 0.1% roflumilast as an active ingredient although the concentration of roflumilast is 1/10. Accordingly, this shows a significant effect of being prepared as a soluble-type eye drop.

3. Test 3

An allergic conjunctivitis inhibitory effect when the amount of polyvinyl alcohol or the pH of eye drop was changed was studied.

3-1. Preparation of test solutions

Example 5

A clear eye drop (viscosity: 4.4 mPa·s) was obtained by carrying out the same procedure as described in Example 4 except that the amount of a 5% aqueous solution of polyvinyl alcohol to be added was changed to 60 g and dibasic sodium phosphate (q.s.) was used instead of borax (q.s.). Hereinafter the resulting solution is designated as Test solution 5.

Example 6

A clear eye drop (viscosity: 2.4 mPa·s) was obtained by carrying out the same procedure as described in Example 4 except that the amount of a 5% aqueous solution of polyvinyl alcohol to be added was changed to 28 g and

dibasic sodium phosphate (q.s.) was used instead of borax (q.s.). Hereinafter the resulting solution is designated as Test solution 6.

Example 7

A clear eye drop (viscosity: 2.4 mPa·s) was obtained by carrying out the same procedure as described in Example 4 except that the pH was adjusted to 5. Hereinafter the resulting solution is designated as Test solution 7.
The Test solutions prepared by the above-mentioned procedures are shown in Table 6, respectively. For the sake of simplifying the table, only roflumilast and a viscosity-increasing agent both of which are necessary components for the comparison and evaluation of the effect of the present invention are shown in the formulation in Table 6.
[Table 6]

Table 6

| Component | concentration (%(w/v)) | | |
|---|---|---|---|
| | Test solution 5 | Test solution 6 | Test solution 7 |
| Roflumilast | 0.01 | 0.01 | 0.01 |
| Polyvinyl alcohol | 3.0 | 1.4 | 1.4 |
| pH | 8 | 8 | 5 |
| Appearance | Clear | Clear | Clear |
| Viscosity (mPa·s) | 4.4 | 2.4 | 2.4 |

3-2. Test Method and Evaluation Method

The test was carried out in the same manner as the method described in 1-2. As a control, a physiological saline solution was used. The evaluation was carried out by the same method as described in 1-3. The inhibition ratios (%) of Test solutions are shown in Table 7 (the case number was 16 or 18 eyes).
[Table 7]

Table 7

| | Inhibition ratio (%) |
|---|---|
| Test solution 5 | 20.7 |
| Test solution 6 | 41.4 |
| Test solution 7 | 31.0 |

3-3. Test Results and Discussion

As is apparent from Table 7, it was confirmed that in all the soluble-type eye drops obtained by formulating polyvinyl alcohol in an eye drop containing 0.01% roflumilast as an active ingredient, the allergic conjunctivitis inhibitory effect of roflumilast is enhanced even if the amount of polyvinyl alcohol or the pH of eye drop was changed.

[Preparation Examples]

The following preparations were obtained in accordance with the preparation method described in the examples. Incidentally, in the following preparation examples, the amount of each component is represented by the content thereof in 100 mL.

Preparation Example 1

| | |
|---|---|
| Roflumilast | 0.01 g |

(continued)

| | |
|---|---|
| Hydroxypropylmethyl cellulose | 0.5 g |
| Polyoxyl 35 castor oil | 5 g |
| Benzalkonium chloride | 0.005 g |
| Disodium edetate | 0.01 g |
| Sodium chloride | q.s. |
| Dibasic sodium phosphate | q.s. |
| 1 N sodium hydroxide | q.s. |
| Hydrochloric acid | q.s. |
| Sterile purified water | q.s. |

In the above formulation, by changing the amount of hydroxypropylmethyl cellulose to 0.1 g, 0.3 g or 1 g, a similar preparation to Preparation Example 1 can be obtained. Further, by changing the amount of roflumilast to 0.0003 g, 0.001 g, 0.005 g or 0.03 g, a similar preparation to Preparation Example 1 can be obtained.

Preparation Example 2

| | |
|---|---|
| Roflumilast | 0.01 g |
| Polyvinyl alcohol | 1 g |
| Polyoxyl 35 castor oil | 5 g |
| Benzalkonium chloride | 0.005 g |
| Disodium edetate | 0.01 g |
| Concentrated glycerin | q.s. |
| Dibasic sodium phosphate | q.s. |
| 1 N sodium hydroxide | q.s. |
| Hydrochloric acid | q.s. |
| Sterile purified water | q.s. |

In the above formulation, by changing the amount of polyvinyl alcohol to 0.1 g, 0.3 g, 0.8 g, 1.4 g, 1.8 g, 2.5 g or 5 g, a similar preparation to Preparation Example 2 can be obtained. Further, by changing the amount of roflumilast to 0.0003 g, 0.001 g, 0.005 g or 0.03 g, a similar preparation to Preparation Example 2 can be obtained.

Preparation Example 3

| | |
|---|---|
| Roflumilast | 0.01 g |
| Polyvinyl alcohol | 1 g |
| Polyoxyl 35 castor oil | 3 g |
| Benzalkonium chloride | 0.01 g |
| Disodium edetate | 0.01 g |
| Concentrated glycerin | q.s. |
| Dibasic sodium phosphate | q.s. |
| Borax | q.s. |
| 1 N sodium hydroxide | q.s. |
| Hydrochloric acid | q.s. |
| Sterile purified water | q.s. |

In the above formulation, by changing the amount of polyvinyl alcohol to 0.1 g, 0.3 g, 0.8 g, 1.4 g, 1.8 g, 2.5 g or 5 g, a similar preparation to Preparation Example 3 can be obtained. Further, by changing the amount of roflumilast to 0.0003 g, 0.001 g, 0.005 g or 0.03 g, a similar preparation to Preparation Example 3 can be obtained.

Preparation Example 4

| | |
|---|---|
| Roflumilast | 0.01 g |
| Polyvinyl alcohol | 1 g |
| Polyoxyl 35 castor oil | 3 g |
| Benzalkonium chloride | 0.005 g |
| Disodium edetate | 0.01 g |
| Concentrated glycerin | q.s. |
| Trometamol | q.s. |
| 1 N sodium hydroxide | q.s. |
| Hydrochloric acid | q.s. |
| Sterile purified water | q.s. |

In the above formulation, by changing the amount of polyvinyl alcohol to 0.1 g, 0.3 g, 0.8 g, 1.4 g, 1.8 g, 2.5 g or 5 g, a similar preparation to Preparation Example 4 can be obtained. Further, by changing the amount of roflumilast to 0.0003 g, 0.001 g, 0.005 g or 0.03 g, a similar preparation to Preparation Example 4 can be obtained.

## Claims

**1.** An eye drop comprising roflumilast as an active ingredient, wherein by formulating at least one type of viscosity-increasing agent therein, the efficacy of roflumilast is enhanced and also the viscosity of the eye drop at 25°C is made 1.5 to 500 mPa·s.

**2.** The eye drop according to claim 1, wherein the viscosity of the eye drop at 25°C is 1.5 to 50 mPa·s.

**3.** The eye drop according to claim 1, wherein the concentration of roflumilast is 0.00001 to 0.05% (w/v).

**4.** The eye drop according to claim 1, wherein the concentration of roflumilast is 0.0001 to 0.03% (w/v).

**5.** The eye drop according to claim 1, wherein the viscosity-increasing agent is hydroxypropylmethyl cellulose, polyvinyl alcohol or carboxyvinyl polymer.

**6.** The eye drop according to claim 1, wherein it is made a soluble type by further formulating a surfactant therein.

**7.** The eye drop according to claim 6, wherein the surfactant is at least one member selected from polysorbate 80, polyoxyethylene hydrogenated castor oil 60, polyoxyl 35 castor oil and polyoxyl 40 monostearate.

**8.** A method in which in an eye drop comprising roflumilast as an active ingredient, by formulating at least one type of viscosity-increasing agent therein, the efficacy of roflumilast is enhanced and also the viscosity of the eye drop at 25°C is made 1.5 to 500 mPa·s.

**9.** A method of treating an eye disease comprising administering a pharmacologically effective amount of an eye drop comprising roflumilast as an active ingredient, in which by formulating at least one type of viscosity-increasing agent therein, the efficacy of roflumilast is enhanced and also the viscosity of the eye drop at 25°C is made 1.5 to 500 mPa·s to a patient.

**10.** The treatment method according to claim 9, wherein the viscosity of the eye drop at 25°C is 1.5 to 50 mPa·s.

**11.** The treatment method according to claim 9, wherein the concentration of roflumilast is 0.00001 to 0.05% (w/v).

**12.** The treatment method according to claim 9, wherein the concentration of roflumilast is 0.0001 to 0.03% (w/v).

**13.** The treatment method according to claim 9, wherein the viscosity-increasing agent is hydroxypropylmethyl cellulose,

polyvinyl alcohol or carboxyvinyl polymer.

**14.** The treatment method according to claim 9, wherein the eye drop is made a soluble type by further formulating a surfactant therein.

**15.** The treatment method according to claim 14, wherein the surfactant is at least one member selected from polysorbate 80, polyoxyethylene hydrogenated castor oil 60, polyoxyl 35 castor oil and polyoxyl 40 monostearate.

**INTERNATIONAL SEARCH REPORT**

International application No.
PCT/JP2006/311576

A. CLASSIFICATION OF SUBJECT MATTER
*A61K31/44*(2006.01)i, *A61K9/08*(2006.01)i, *A61K47/32*(2006.01)i, *A61K47/34*(2006.01)i, *A61K47/38*(2006.01)i, *A61P27/02*(2006.01)i

According to International Patent Classification (IPC) or to both national classification and IPC

B. FIELDS SEARCHED

Minimum documentation searched (classification system followed by classification symbols)
A61K31/44, A61K9/08, A61K47/32, A61K47/34, A61K47/38, A61P27/02

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | WO 2003/099278 A1 (ALTANA PHARMA AG.), 04 December, 2003 (04.12.03), Full text; particularly, Claims; page 3, line 32 to page 4, line 20; example 3 & EP 1511481 A1 & JP 2005-529928 A | 1-8 |
| Y | WO 2003/099334 A1 (ALTANA PHARMA AG.), 04 December, 2003 (04.12.03), Full text; particularly, Claims; page 6, 10 to the last line & EP 1511516 A1 & JP 2005-529930 A | 1-8 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 August, 2006 (07.08.06) | 15 August, 2006 (15.08.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

International application No.
PCT/JP2006/311576

C (Continuation). DOCUMENTS CONSIDERED TO BE RELEVANT

| Category* | Citation of document, with indication, where appropriate, of the relevant passages | Relevant to claim No. |
|---|---|---|
| Y | JP 7-506579 A (Sando Yakuhin Kabushiki Kaisha), 20 July, 1995 (20.07.95), Full text; particularly, page 2, lower left column, line 27 to page 3, upper right column, line 18; examples 1 to 5 & WO 1993/023010 A1 & EP 642332 A1 & US 5951971 A | 1-8 |

Form PCT/ISA/210 (continuation of second sheet) (April 2005)

**INTERNATIONAL SEARCH REPORT**

International application No.
PCT/JP2006/311576

**Box No. II Observations where certain claims were found unsearchable (Continuation of item 2 of first sheet)**

This international search report has not been established in respect of certain claims under Article 17(2)(a) for the following reasons:

1. [X] Claims Nos.: 9-15
because they relate to subject matter not required to be searched by this Authority, namely:
Claims 9 to 15 pertain to methods for treatment of the human body by therapy.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the

[ ] The additional search fees were accompanied by the applicant's protest and, where applicable, payment of a protest fee..

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 8512041 T
- WO 03099278 A
- WO 03099334 A